# EUROPEAN PATENT APPLICATION

(11) **EP 2 702 995 A1**
(43) Date of publication of application: **05.03.2014**
(21) Application number: 12777027.9
(22) Date of filing: 20.04.2012
(51) Int. Cl.: A61K 31/4545, A61K 9/20, A61K 47/10, A61K 47/26, A61K 47/32, A61K 47/36, A61K 47/38, A61K 47/40

(54) **RAPIDLY DISSOLVING ORAL TABLET**

(30) Priority: 28.04.2011 JP 2011100687
(71) Applicant: Mitsubishi Tanabe Pharma Corporation, Osaka-shi Osaka 541-8505 (JP)
(72) Inventor: NARISAWA, Shinji, Osaka 541-8505 (JP); SUGIMOTO, Masaaki, Osaka 541-8505 (JP); KITAOKA, Kenichi, Osaka 541-8505 (JP); OOKAWA, Akiko, Osaka 541-8505 (JP); MORIMOTO, Masayuki, Osaka 541-8505 (JP)
(74) Representative: HOFFMANN EITLE
(86) International application number: PCT/JP2012/060754
(87) International publication number: WO 2012/147660

(57) **Abstract**

Provided is an orally disintegrating tablet comprising a bitter-tasting pharmaceutically active ingredient in the form of bepotastine or a pharmacologically acceptable salt thereof that enables the bitter taste thereof to be masked, has both superior oral cavity disintegration and adequate hardness, and can be produced with ordinary tablet production equipment. The present invention relates to an orally disintegrating tablet containing bepotastine or a pharmacologically acceptable salt thereof, menthol or cyclodextrin, a water-insoluble polymer, and a disintegrating agent; and a method for producing an orally disintegrating tablet, comprising: 1) a step for obtaining granulated granules by mixing and granulating bepotastine or a pharmacologically acceptable salt thereof, a water-insoluble polymer, and optionally a vehicle, 2) a step for obtaining granules for tableting by mixing the granulated granules, menthol, a disintegrating agent, and optionally a lubricant and/or sweetener, and 3) a step for compressing the granules for tableting.

## Description

### TECHNICAL FIELD

The present invention relates to an orally disintegrating tablet comprising bepotastine or a pharmacologically acceptable salt thereof that masks its bitter taste while also having adequate hardness.

### BACKGROUND ART

The providing of a tablet that can be easily taken by the elderly and patients with dysphagia is thought to lead to improvement of quality of life as well as improvement of drug compliance. Since orally disintegrating tablets in particular are not only easy to take, but also increase the potential for the launching of new products by the demonstration of their bioequivalency with previously approved preparations, development is proceeding on numerous products. In addition, since orally disintegrating tablets also have the advantage of reducing formulation work in the clinical site such as crushing and suspending, the advantage leads to demand for such an orally disintegrating tablet. However, since there are many drugs that have an unpleasant taste such as bitter or sour taste, technologies for suppressing such unpleasant taste are important for enabling such drugs to be formulated into orally disintegrating tablets.

Although bepotastine besilate is used as a selective histamine 1 receptor antagonist for conditions such as allergic rhinitis and hives, it is known to have the problem of having an intensely bitter taste. Consequently, it is necessary to mask this bitter taste in order to administer orally.

With respect to masking the bitter taste of drugs, International Publication No. WO 2009/054432 (Patent Document 1), for example, discloses that an orally disintegrating tablet is obtained in which bitter taste is masked by mixing with cetirizine or salt thereof, which is known to have an intensely bitter taste, and cyclodextrin, and mixing additionally with cornstarch and cellulose followed by direct powder compression. However, since the cornstarch and cellulose are highly hygroscopic, there is the shortcoming of the tablet being susceptible to decreases in hardness during storage.

In addition, International Publication No. WO 2007/011018 (Patent Document 2) discloses that the bitter taste of drugs when disintegrating in the mouth can be effectively suppressed by using granules that comprise a bitter-tasting drug and a vehicle and for which bitter taste is not suppressed, and granules comprising a water-soluble sugar, for production of an orally disintegrating tablet. However, since the production process of the orally disintegrating tablet disclosed in Patent Document 2 requires more production steps in comparison with ordinary production processes, it has the shortcoming of poor production efficiency. In addition, there is also concern over a decrease in content uniformity attributable to granule segragation.

International Publication No. WO 2005/094812 (Patent Document 3) discloses a pharmaceutical composition having as an active ingredient thereof a bitter-tasting component in the form of nateglinide, wherein the bitter taste thereof is reduced by containing at least one of an organic acid or inorganic acid, sweetener and fragrance, and lists menthol as an example of a fragrance. However, in an example that incorporates menthol, there is a problem of the presence of an aftertaste, thus the effect of masking bitter taste is not adequate.

Japanese Unexamined Patent Publication No. 2005-343800 (Patent Document 4) discloses that an effect of masking the bitter taste of caffeine granules is obtained by using coated caffeine granules in an orally disintegrating tablet that are obtained by coating caffeine granules having specific physical properties with a coating agent comprising a poorly water soluble polymer compound and a plasticizer. However, the bitter taste masking effect is only described with respect to caffeine granules having specific physical properties (such as particle diameter or hardness). In addition, there is also the problem that particles having an average primary particle diameter of 200 µm to 600 µm being unpleasant (rough) are remained on the tongue when taking.

On the other hand, with respect to technologies for incorporating cyclic polysaccharides such as cyclodextrin in an orally disintegrating tablet, International Publication No. WO 2005/004923 (Patent Document 5), for example, discloses an orally disintegrating tablet comprising an active ingredient and cyclodextrin or a cyclodextrin derivative, wherein cyclodextrin or a cyclodextrin derivative constitutes 70% by mass or more of the components in the tablet. However, components incorporating 70% by weight or more of cyclodextrin have the shortcoming of being unpleasant (rough feeling on the tongue) when taking. In addition, since cyclodextrin is highly hygroscopic, in the case of incorporating a large amount thereof in formulated components, there is concern over a decrease in hardness of the tablet during storage.

In addition, Japanese Unexamined Patent Publication No. 2005-298338 (Patent Document 6) discloses a rapidly disintegrating compression tablet that comprises a pharmaceutical component and a cyclic oligosaccharide that is not substituted with hydrophilic functional groups (cyclodextrin). However, there is no description regarding masking of bitter taste.

International Publication No. WO 2010/061846 (Patent Document 7) discloses an orally disintegrating tablet that combines a vehicle having favorable wettability with respect to water, a water-insoluble polymer and a disintegrating agent, and is characterized by being able to be produced with ordinary tablet production equipment, has hardness that is adequate in terms of practical use, and is resistant to changes in properties caused by factors such as humidity, and it is also disclosed that this orally disintegrating tablet incorporates cyclodextrin as a solubilizing assistant. However, there is no description regarding application to drugs having a bitter taste, such as bepotastine or a pharmacologically acceptable salt thereof.

In addition, although technologies for incorporating menthol in orally disintegrating tablets are disclosed in the aforementioned Patent Documents 1, 2, 4, 6 and 7, the menthol is merely incorporated as a refreshing agent, fragrance or sleepiness preventive agent.

### PRIOR ART DOCUMENTS

### Patent Documents

Patent Document 1: International Publication No. WO 2009/054432
Patent Document 2: International Publication No. WO 2007/011018
Patent Document 3: International Publication No. WO 2005/094812
Patent Document 4: Japanese Unexamined Patent Publication No. 2005-343800
Patent Document 5: International Publication No. WO 2005/004923
Patent Document 6: Japanese Unexamined Patent Publication No. 2005-298338
Patent Document 7: International Publication No. WO 2010/061846

### DISCLOSURE OF THE INVENTION

### Problems to be Solved by the Invention

With the foregoing in view, there is a desire for the development of an orally disintegrating tablet comprising a bitter-tasting pharmaceutically active ingredient in the form of bepotastine or pharmacologically acceptable salt thereof that enables the bitter taste thereof to be masked, has both superior oral cavity disintegration and adequate hardness, and can be produced with ordinary tablet production equipment.

### Means for Solving the Problems

As a result of conducting extensive studies to solve the aforementioned problems, the inventor of the present invention found that by containing 1) a bitter-tasting pharmacologically active ingredient in the form of bepotastine or pharmacologically acceptable salt, 2) a bitter taste masking agent in the form of menthol or cyclodextrin, and 3) a water-insoluble polymer and disintegrating agent, an orally disintegrating tablet is obtained that can be produced with ordinary tablet production equipment, has adequate hardness capable of withstanding production and distribution processes, has superior oral cavity disintegration, and masks the bitter taste of the bepotastine or pharmacologically acceptable salt thereof, thereby leading to completion of the present invention.

The present invention relates to an orally disintegrating tablet comprising bepotastine or a pharmacologically acceptable salt thereof, menthol or cyclodextrin, a water-insoluble polymer, and a disintegrating agent.

The present invention also relates to a method for producing the orally disintegrating tablet, comprising:
1) a step for obtaining granulated granules by mixing and granulating bepotastine or a pharmacologically acceptable salt thereof, a water-insoluble polymer, and optionally a vehicle,
2) a step for obtaining granules for tableting by mixing the granulated granules, menthol, a disintegrating agent, and optionally a lubricant and/or sweetener, and
3) a step for compressing the granules for tableting.

The present invention also relates to a method for producing the orally disintegrating tablet, comprising:
1) a step for obtaining granulated granules by mixing and granulating bepotastine or a pharmacologically acceptable salt thereof, cyclodextrin, a water-insoluble polymer, and optionally a vehicle,
2) a step for obtaining granules for tableting by mixing the granulated granules, a disintegrating agent, and optionally a lubricant and/or sweetener, and
3) a step for compressing the granules for tableting.

Moreover, the present invention further relates to an orally disintegrating tablet incorporating granulated granules obtained by mixing and granulating bepotastine or a pharmacologically acceptable salt thereof, a water-insoluble polymer, and optionally a vehicle; an orally disintegrating tablet incorporating granulated granules obtained by granulating bepotastine or a pharmacologically acceptable salt thereof and a water-insoluble polymer by wet granulation; and granulated granules obtained by granulating bepotastine or a pharmacologically acceptable salt thereof and a water-insoluble polymer by wet-granulation.

### Effects of the Invention

The orally disintegrating tablet of the present invention masks the bitter taste of bepotastine or a pharmacologically acceptable salt thereof, has both superior oral cavity disintegration and adequate hardness, and can be produced with ordinary tablet production equipment.

### BEST MODE FOR CARRYING OUT THE INVENTION

As has been described above, the present invention relates to an orally disintegrating tablet comprising bepotastine or a pharmacologically acceptable salt thereof, menthol or cyclodextrin, a water-soluble polymer, and a disintegrating agent.

The bepotastine or pharmacologically acceptable salt thereof contained in the orally disintegrating tablet of the present invention is a known compound represented by the following formula: 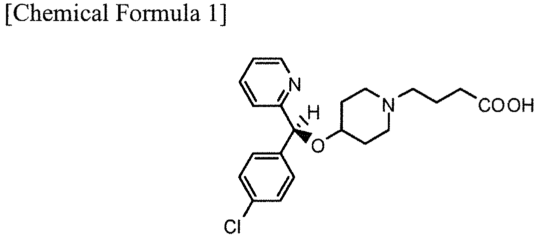 or a pharmacologically acceptable salt thereof, and is used as a selective histamine 1 receptor antagonist for the treatment of allergic rhinitis, hives, and the like.

As the pharmacologically acceptable salt of bepotastine, an addition salt with a pharmacologically acceptable acid or a carbonate with a pharmacologically acceptable metal can be used. Examples of such salts that can be used preferably include addition salts of inorganic acids (such as sulfates, hydrochlorides or phosphates), addition salts of organic acids (such as succinates, maleates, methanesulfonates, benzenesulfonates or benzoates), and carbonates of alkali metals (such as sodium, potassium or lithium) and alkaline earth metals (such as magnesium, calcium or barium). Among them, addition salts of benzenesulfonic acid, namely bepotastine besilate, and addition salts of benzoic acid can be used particularly preferably, and bepotastine besilate can be used most preferably.

In the orally disintegrating tablet of the present invention, in the case of incorporating bepotastine or bepotastine besilate as a pharmacologically acceptable salt thereof, although bepotastine besilate can be incorporated in a desired amount, it can be incorporated at, for example, 1 to 20 mg, preferably 2.5 to 15 mg, and more preferably 5 or 10 mg. Bepotastine or a pharmacologically acceptable salt thereof can be incorporated at 1 to 5 parts by weight, and preferably 3 to 5 parts by weight, based on a total of 100 parts by weight of the orally disintegrating tablet.

The menthol incorporated in the orally disintegrating tablet of the present invention is 1-menthol. In the orally disintegrating tablet of the present invention, the menthol can be incorporated in an amount within an arbitrary range capable of suppressing the bitter taste of bepotastine or a pharmacologically acceptable salt thereof. More specifically, the menthol can be incorporated at a mass ratio within the range of 1:0.01 to 1, preferably within a range of 1:0.05 to 0.5, more preferably within a range of 1:0.1 to 0.5, particularly preferably within a range of 1:0.1 to 0.4, and most preferably within a range of 1:0.1 to 0.3, with respect to the bepotastine or a pharmacologically acceptable salt thereof.

The cyclodextrin incorporated in the orally disintegrating tablet of the present invention is α-cyclodextrin, β-cyclodextrin or γ-cyclodextrin, and these are cyclic molecules containing 6, 7 and 8 glucose units, respectively. In the orally disintegrating tablet of the present invention, as a result of incorporating cyclodextrin in addition to suppressing the bitter taste of bepotastine or a pharmacologically acceptable salt thereof, the hardness of the preparation is improved. α-Cyclodextrin, β-cyclodextrin or γ-cyclodextrin can be used alone or as a mixture. In particular, β-cyclodextrin can be used preferably.

In the orally disintegrating tablet of the present invention, the cyclodextrin can be incorporated in an amount within an arbitrary range capable of suppressing the bitter taste of bepotastine or a pharmacologically acceptable salt thereof. More specifically, the cyclodextrin can be incorporated at a mass ratio within the range of, for example, 1:3 to 1:30, preferably within the range of 1:3 to 20, more preferably within the range of 1:3 to 10, particularly preferably within the range of 1:4 to 6, and most preferably within the range of 1:5, with respect to the bepotastine or a pharmacologically acceptable salt thereof.

The water-insoluble polymer incorporated in the orally disintegrating tablet of the present invention refers to an arbitrary water-insoluble polymer having an action of a tablet binder and an action that does not allow a decrease in wettability of a vehicle. Incorporating a water-insoluble polymer in the orally disintegrating tablet allows the tablet to rapidly disintegrate in the mouth by allowing water to rapidly permeate into the tablet.

Examples of such water-insoluble polymers include hydroxypropyl methylcellulose acetate succinate (e.g. HPMC-AS, Shin-Etsu Chemical Co., Ltd.), methacrylic acid copolymer S (e.g. Eudragit S, Roehm GmbH), methacrylic acid copolymer L (e.g. Eudragit L, Roehm GmbH), carboxymethyl ethyl cellulose (e.g. CMEC, Sanyo Chemical Industries, Ltd.), ethyl cellulose (e.g. Ethocell, Dow Chemical Co.), hydroxypropyl methylcellulose phthalate (e.g. HP-50, Shin-Etsu Chemical Co., Ltd.) and aminomethacrylate copolymer (e.g. Eudragit RS or RL, Roehm GmbH), preferably hydroxypropyl methylcellulose acetate succinate, methacrylic acid copolymer S, carboxymethyl ethyl cellulose and ethyl cellulose, and more preferably hydroxypropyl methylcellulose acetate succinate.

In the orally disintegrating tablet of the present invention, the aforementioned water-insoluble polymers can be respectively incorporated alone or as a mixture of two or more types of water-insoluble polymers can be incorporated in combination.

In the orally disintegrating tablet of the present invention, the water-insoluble polymer can be incorporated within the range of, for example, 1 to 30 parts by weight, preferably within the range of 1 to 25 parts by weight, and more preferably within the range of 3 to 20 parts by weight, based on a total of 100 parts by weight of the tablet. Furthermore, the inventors of the present invention found that the oral cavity disintegration time tends to become shorter accompanying an increase in the incorporated amount of the water-insoluble polymer in the orally disintegrating tablet of the present invention.

The disintegrating agent incorporated in the orally disintegrating tablet of the present invention refers to an arbitrary disintegrating agent commonly used in pharmaceutical preparations. The tablet disintegrates more rapidly in the mouth by incorporating the disintegrating agent in the orally disintegrating tablet along with the water-insoluble polymer. Examples of such disintegrating agents that can be used include celluloses such as carboxymethyl cellulose calcium, lowly substituted hydroxypropyl cellulose, carboxymethyl cellulose, croscarmellose sodium or crystalline cellulose, starches such as cornstarch, partially α-modified starch or sodium carboxymethyl starch, and crospovidone. Among them, preferable examples include croscarmellose sodium, carboxymethyl cellulose and crospovidone, while more preferable examples include croscarmellose sodium and carboxymethyl cellulose.

In the orally disintegrating tablet of the present invention, the aforementioned disintegrating agents can be respectively incorporated alone or two or more types of disintegrating agents can be incorporated in combination.

In the orally disintegrating tablet of the present invention, the disintegrating agent can be incorporated, for example, within the range of 1 to 20 parts by weight, preferably within the range of 1 to 10 parts by weight, and more preferably within the range of 1 to 5 parts by weight, based on a total of 100 parts by weight of the tablet.

In the orally disintegrating tablet of the present invention, a vehicle can be further incorporated in addition to the bepotastine or a pharmacologically acceptable salt thereof, menthol or cyclodextrin, water-insoluble polymer and disintegrating agent.

The vehicle is an arbitrary vehicle commonly used in pharmaceutical preparations. Examples of such vehicles include sugar-alcohols or sugars such as mannitol, xylitol, erythritol, maltitol, sorbitol, lactose, sucrose, maltose or trehalose, preferable examples include mannitol, erythritol and lactose, and mannitol is particularly preferable. In addition, a vehicle having favorable wettability with respect to water can be used preferably for the vehicle. Here, a vehicle applicable to the following definition corresponds to a vehicle having favorable wettability with respect to water. Namely, when wetting time with respect to water of a tablet obtained by compressing a vehicle at a tableting pressure of 1000 kg using a flat pestle having a diameter of 10 mm is measured according to the procedure described in International Publication No. WO 2010/061846, that vehicle corresponds to a vehicle having favorable wettability with respect to water in the case that wetting time is within 300 seconds. Examples of vehicles having favorable wettability with respect to water as described above include one or more types of sugar-alcohols or sugars selected from the group consisting of mannitol, erythritol and xylitol, and particularly preferably mannitol.

In the orally disintegrating tablet of the present invention, the aforementioned vehicles may respectively be incorporated alone or two or more types of vehicles can be incorporated in combination.

In the orally disintegrating tablet of the present invention, the vehicle can be incorporated, for example, within the range of 30 to 95 parts by weight, preferably within the range of 40 to 95 parts by weight, and more preferably within the range of 50 to 95 parts by weight, based on a total of 100 parts by weight of the tablet.

In the orally disintegrating tablet of the present invention, various types of additives ordinarily incorporated in orally disintegrating tablets can be further incorporated in addition to the aforementioned components. Examples of such additives include lubricants such as magnesium stearate, sodium stearyl fumarate, talc, light silicic anhydride, hydrated silicon dioxide, alkaline earth metal stearates (such as magnesium stearate or calcium stearate), sucrose higher fatty acid esters or glycerin higher fatty acid esters, and sweeteners such as aspartame, saccharin, sodium saccharin, potassium acesulfame, sucralose, stevia or thaumatin. Moreover, colorants such as edible red dye nos. 2 and 3, edible yellow dye nos. 4 and 5, edible blue dye nos. 1 and 2, aluminum lakes thereof, iron sesquioxide or yellow iron sesquioxide, correctives such as sodium chloride, sodium citrate, sodium glutamate or sodium bicarbonate, pH adjusters such as citrates or carbonates, solubilizing agents such as sodium lauryl sulfate or sorbitan monostearate, solubilizing assistants such as arginine or lysine, and fragrances such as peppermint oil or strawberry essence, can also be further incorporated.

A preferable aspect of the blending ratios of each component incorporated in the orally disintegrating tablet of the present invention consists of 3 to 5 parts by weight of bepotastine or a pharmacologically acceptable salt thereof, 0.1 to 1 part by weight of 1-menthol, 5 to 25 parts by weight of the water-insoluble polymer, 1 to 5 parts by weight of the disintegrating agent, and 65 to 90 parts by weight of the vehicle based on a total of 100 parts by weight of the tablet.

Another preferable aspect of the blending ratios of each component incorporated in the orally disintegrating tablet of the present invention consists of 3 to 5 parts by weight of bepotastine or a pharmacologically acceptable salt thereof, 15 to 25 parts by weight of cyclodextrin, 3 to 15 parts by weight of the water-insoluble polymer, 2 to 5 parts by weight of the disintegrating agent, and 55 to 75 parts by weight of the vehicle based on a total of 100 parts by weight of the tablet.

The orally disintegrating tablet of the present invention can be produced with ordinary tablet production equipment without using special tablet production equipment by an ordinary method for producing orally disintegrating tablets consisting of incorporating bepotastine or a pharmacologically acceptable salt thereof, menthol or cyclodextrin, a water-insoluble polymer and a disintegrating agent.

The orally disintegrating tablet of the present invention that comprises menthol can be produced by, for example, a method comprising the following steps:
A-1) a step for obtaining granulated granules by mixing and granulating bepotastine or a pharmacologically acceptable salt thereof, a water-insoluble polymer, and optionally a vehicle,
A-2) a step for obtaining granules for tableting by mixing the granulated granules, menthol, a disintegrating agent, and optionally a lubricant and/or sweetener, and
A-3) a step for compressing the granules for tableting.

1) In the step for obtaining granulated granules of the aforementioned step A-1), bepotastine or a pharmaceutical acceptable salt thereof, a water-insoluble polymer, and optionally a vehicle, are first sequentially mixed. Although an arbitrary method can be used for the mixing method, a commonly used method using, for example, a double cone mixer, fluidized bed granulator or high-speed mixer granulator can be used. After mixing, the mixture can be sifted as necessary using, for example, a 22 mesh sieve as defined in the Japanese Pharmacopoeia.

A dry granulation method or wet granulation method, for example, can be used in the next granulation step. In the case of dry granulation method, desired granules can be obtained by granulating a powder mixture of the aforementioned components using a roller compactor or roller granulator and the like. In the case of wet granulation method, desired granulated granules can be obtained by granulating while adding water to a powder mixture of the aforementioned components by means such as spraying while the mixture is allowed to flow using a fluidized bed granulator or high-speed mixer granulator and the like, followed by drying the resulting granulation product. In another method used in the case of wet granulation, desired granulated granules can be obtained by granulating while adding a solution of a water-insoluble polymer (e.g. an ethanol solution or ethanol/aqueous solution) to a powder mixture comprising bepotastine or a pharmacologically acceptable salt thereof and optionally a vehicle by means such as spraying while the mixture is allowed to flow using a fluidized bed granulator or high-speed mixer granulator and the like, followed by drying the resulting granulation product.

2) Granules for tableting can be obtained by mixing menthol, a disintegrating agent, and optionally a lubricant and/or sweetener, as well as additives such as a colorant, corrective, pH adjuster, solubilizing agent, solubilizing assistant or fragrance as necessary, into the granulated granules obtained in this manner.

3) Compression of the granules for tableting obtained in this manner can be carried out using a commonly used tableting machine such as a single punch tableting machine or rotary tableting machine. Although tableting pressure can be suitably selected depending on the hardness and other properties of the target tablet, it can be about 10 to 5000 kg/pestle, preferably about 20 to 4000 kg/pestle, and particularly preferably about 100 to 2000 kg/pestle. In the case of using, for example, a compaction analyzer manufactured by Kikusui Seisakusho Ltd. for the tableting machine, tableting is preferably carried out at a tableting pressure of 400 to 1000 kg/pestle in the case of using, for example, a pestle having a diameter of 9.5 mm.

Furthermore, in the case of incorporating menthol, after first separately preparing a preliminary corrective preparation comprising menthol, the preliminary corrective preparation is mixed with the granulated granules, a disintegrating agent, and optionally a lubricant and/or sweetener, and the resulting mixture can be used in the subsequent steps. An example of a method for preparing this type of menthol-comprising preliminary corrective preparation includes addition of a lubricant such as hydrated silicon dioxide and a vehicle such as mannitol to 1-menthol, and optionally further adding a fragrance such as peppermint oil. Another example of a method for preparing the menthol-comprising preliminary corrective preparation includes mixing and granulation of 1-menthol, a vehicle such as mannitol, and optionally a fragrance such as peppermint oil, followed by further adding a lubricant such as hydrated silicon dioxide and granulating.

Accordingly, the present invention also relates to a method for producing an orally disintegrating tablet that comprises the aforementioned steps; and an orally disintegrating tablet produced according to a method comprising the aforementioned steps, namely an orally disintegrating tablet produced according to a method comprising:
A-1) a step for obtaining granulated granules by mixing and granulating bepotastine or a pharmacologically acceptable salt thereof, a water-insoluble polymer, and optionally a vehicle,
A-2) a step for obtaining granules for tableting by mixing the granulated granules, menthol, a disintegrating agent, and optionally a lubricant and/or sweetener, and
A-3) a step for compressing the granules for tableting.

As a specific example thereof, the present invention also relates to an orally disintegrating tablet produced according to a method comprising:
A-1) a step for obtaining granulated granules by mixing and granulating bepotastine or a pharmacologically acceptable salt thereof, a water-insoluble polymer, and optionally a vehicle by a wet granulation method,
A-2-1) a step for obtaining a menthol-comprising preliminary corrective preparation by adding a lubricant, a vehicle and optionally further adding a fragrance, to 1-menthol,
A-2-2) a step for obtaining granules for tableting by mixing the granulated granules, the menthol-containing preliminary corrective preparation, a disintegrating agent, and optionally a lubricant and/or sweetener, and
A-3) a step for compressing the granules for tableting.

As another specific example thereof, the present invention also relates to an orally disintegrating tablet produced according to a method comprising:
A-1) a step for obtaining granulated granules by mixing and granulating bepotastine or a pharmacologically acceptable salt thereof, a water-insoluble polymer, and optionally a vehicle,
A-2-1) a step for obtaining a menthol-containing preliminary corrective preparation by mixing and granulating 1-menthol, a vehicle and optionally a fragrance, followed by further adding a lubricant and granulating,
A-2-2) a step for obtaining granules for tableting by mixing the granulated granules, the menthol-containing preliminary corrective preparation, a disintegrating agent, and optionally a lubricant and/or sweetener, and
A-3) a step for compressing the granules for tableting.

In addition, an orally disintegrating tablet of the present invention that comprises cyclodextrin can be produced by, for example, a method comprising the following steps:
B-1) a step for obtaining granulated granules by mixing and granulating bepotastine or a pharmacologically acceptable salt thereof, cyclodextrin, a water-insoluble polymer, and optionally a vehicle,
B-2) a step for obtaining granules for tableting by mixing the granulated granules, a disintegrating agent, and optionally a lubricant and/or sweetener, and
B-3) a step for compressing the granules for tableting.

1) In the step for obtaining granulated granules of the aforementioned step B-1), bepotastine or a pharmacologically acceptable salt thereof, cyclodextrin, a water-insoluble polymer, and optionally a vehicle, are first sequentially mixed. Although an arbitrary method can be used for the mixing method, a commonly used method using, for example, a double cone mixer, fluidized bed granulator or high-speed mixer granulator can be used. After mixing, the mixture can be sifted as necessary using, for example, a 22 mesh sieve as defined in the Japanese Pharmacopoeia.

A dry granulation method or wet granulation method, for example, can be used in the next granulation step. In the case of dry granulation, desired granules can be obtained by granulating a powder mixture of the aforementioned components using a roller compactor or roll granulator and the like. In the case of wet granulation, desired granulated granules can be obtained by granulating while adding water to a powder mixture of the aforementioned components by means such as spraying while the mixture is allowed to flow using a fluidized bed granulator or high-speed mixer granulator and the like, followed by drying the resulting granulation product. In another method used in the case of wet granulation, desired granulated granules can be obtained by granulating while adding a solution of a water-insoluble polymer (e.g. an ethanol solution or ethanol/aqueous solution) to a powder mixture consisting of bepotastine or a pharmacologically acceptable salt thereof, cyclodextrin and optionally a vehicle by means such as spraying while the mixture is allowed to flow using a fluidized bed granulator or high-speed mixer granulator and the like, followed by drying the resulting granulation product.

2) Granules for tableting can be obtained by mixing a disintegrating agent, and optionally a lubricant and/or sweetener, as well as additives such as a colorant, corrective, pH adjuster, solubilizing agent, solubilizing assistant, fragrance or corrective as necessary, into the granulated granules obtained in this manner.

3) Compression of the granules for tableting obtained in this manner can be carried out using a commonly used tableting machine such as a single punch tableting machine or rotary tableting machine. Although tableting pressure can be suitably selected depending on the properties of the target tablet such as hardness, it can be about 10 to 5000 kg/pestle, preferably about 20 to 4000 kg/pestle, and particularly preferably about 100 to 2000 kg/pestle. In the case of using, for example, a compaction analyzer manufactured by Kikusui Seisakusho Ltd. for the tableting machine, tableting is preferably carried out at a tableting pressure of 200 to 600 kg/pestle in the case of using, for example, a pestle having a diameter of 10 mm.

Furthermore, in the aforementioned step B-1), the granules for tableting can also be obtained by mixing cyclodextrin, a disintegrating agent, and optionally a lubricant and/or sweetener, along with various types of additives as necessary, into the granulated granules when preparing the granules for tableting in step 2) instead of incorporating the cyclodextrin during preparation of the granulated granules.

Accordingly, the present invention also relates to a method for producing an orally disintegrating tablet that comprises the aforementioned steps; and an orally disintegrating tablet produced according to a method comprising the aforementioned steps, namely an orally disintegrating tablet produced according to a method comprising:
B-1) a step for obtaining granulated granules by mixing and granulating bepotastine or a pharmacologically acceptable salt thereof, cyclodextrin, a water-insoluble polymer, and optionally a vehicle,
B-2) a step for obtaining granules for tableting by mixing the granulated granules, a disintegrating agent, and optionally a lubricant and/or sweetener, and
B-3) a step for compressing the granules for tableting.

The orally disintegrating tablet of the present invention which can be produced with a method like that described above is able to mask the bitter taste of bepotastine or a pharmacologically acceptable salt thereof, has both superior oral cavity disintegration and adequate hardness, and can be produced with ordinary tablet production equipment.

Although varying according to the shape, size and types, incorporated amounts and weights of incorporated components, the tablet hardness of the orally disintegrating tablet of the present invention which can be produced with a method like that described above is normally 15 to 90 N, preferably 20 to 80 N, more preferably 25 to 60 N, and particularly preferably 40 to 60 N, in the case of, for example, a round tablet having a diameter of 5 to 10 mm (weight: 100 to 300 mg).

In addition, although varying according to the shape, size and types, incorporated amounts and weights of incorporated components, the oral cavity disintegration time of the orally disintegrating tablet of the present invention which can be produced with a method like that described above is normally within 50 seconds (5 to 50 seconds), preferably within 45 seconds (5 to 45 seconds), more preferably 5 to 40 seconds, and particularly preferably 5 to 35 seconds.

### Examples

The following provides a more detailed explanation of embodiments of the present invention by way of examples thereof. Furthermore, tablet hardness, oral cavity disintegration time and bitter taste masking were measured as described below.

### Hardness Test Method

The hardness of the orally disintegrating tablet of the present invention was measured using a tablet hardness tester manufactured by Schleuniger GmbH (Type 8M). Measurements were performed three times and the average value thereof was used as the hardness of the tablet.

### Oral Cavity Disintegration Time Measurement Method

The oral cavity disintegration time of the orally disintegrating tablet of the present invention was measured as the amount of time until the tablet was completely disintegrated by saliva when one tablet each was contained in the mouths of healthy adult men (3 subjects) without drinking water to take the tablet and with the tablet remaining still in the mouth (in the absence of actions such as chewing or moving vigorously with the tongue), followed by calculating the average of those times.

### Bitter Taste Masking Evaluation Method

One orally disintegrating tablet of the present invention was placed in the mouth, and masking of bitter taste was evaluated to one of the four grades indicated below (number of subjects: 3).
⊚: No bitter taste
○: Bitter taste suppressed
Δ: Bitter taste slightly suppressed
×: Bitter taste

### Example 1

(1) After mixing 13 g of bepotastine besilate, 65 g of β-cyclodextrin, 288.6 g of D-mannitol and 11.7 g of hydroxypropyl methylcellulose acetate succinate (grade: HF) in a plastic bag, the mixture was sifted with a 22 mesh sieve (pore size: 710 µm) as defined in the Japanese Pharmacopoeia. The resulting sifted product was charged into a fluidized bed granulator (Powrex Corp., MP-01/03) followed by granulating while spraying with 200 g of purified water at a supply air temperature of 50°C over about 50 minutes. The granulation product was dried until the temperature thereof reached 35°C or higher after which the dried product was completely sifted with a 22 mesh sieve (pore size: 710 µm) as defined in the Japanese Pharmacopoeia to obtain granulated granules.
(2) 5.82 g of the aforementioned granulated granules of (1), 0.12 g of crospovidone, 0.06 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 400 kg/pestle) to a weight of 303 mg per tablet to obtain an orally disintegrating tablet (hardness: 56 N, oral cavity disintegration time: 28 seconds). In addition, as a result of investigating the ease at which the tablets can be taken with respect to taste, all three subjects did not perceive any bitter taste (bitter taste masking evaluation result: ⊚).

### Example 2

(1) After mixing 0.3 g of bepotastine besilate, 3 g of β-cyclodextrin, 5.16 g of D-mannitol and 0.27 g of hydroxypropyl methylcellulose acetate succinate (grade: HF) in a plastic bag, the mixture was sifted with a 22 mesh sieve (pore size: 710 µm) as defined in the Japanese Pharmacopoeia. 0.5 g of purified water was added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 5.82 g of the aforementioned granulated granules of (1), 0.12 g of crospovidone, 0.06 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 300 kg/pestle) to a weight of 303 mg per tablet to obtain orally disintegrating tablets (hardness: 49 N, oral cavity disintegration time: 34 seconds). In addition, as a result of investigating the ease at which the tablets can be taken with respect to taste, all three subjects did not perceive any bitter taste (bitter taste masking evaluation result: ⊚).

### Example 3

(1) After mixing 0.3 g of bepotastine besilate, 6 g of β-cyclodextrin, 2.16 g of D-mannitol and 0.27 g of hydroxypropyl methylcellulose acetate succinate (grade: HF) in a plastic bag, the mixture was sifted with a 22 mesh sieve (pore size: 710 µm) as defined in the Japanese Pharmacopoeia. 1 g of purified water was added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 5.82 g of the aforementioned granulated granules of (1), 0.12 g of crospovidone, 0.06 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 250 kg/pestle) to a weight of 303 mg per tablet to obtain orally disintegrating tablets (hardness: 47 N, oral cavity disintegration time: 45 seconds). In addition, as a result of investigating the ease at which the tablets can be taken with respect to taste, all three subjects did not perceive any bitter taste (bitter taste masking evaluation result: ⊚).

### Example 4

(1) After mixing 0.3 g of bepotastine besilate, 8.16 g of β-cyclodextrin and 0.27 g of hydroxypropyl methylcellulose acetate succinate (grade: HF) in a plastic bag, the mixture was sifted with a 22 mesh sieve (pore size: 710 µm) as defined in the Japanese Pharmacopoeia. 1 g of purified water was added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 5.82 g of the aforementioned granulated granules of (1), 0.12 g of crospovidone, 0.06 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 300 kg/pestle) to a weight of 303 mg per tablet to obtain orally disintegrating tablets (hardness: 55 N, oral cavity disintegration time: 42 seconds). In addition, as a result of investigating the ease at which the tablets can be taken with respect to taste, all three subjects did not perceive any bitter taste (bitter taste masking evaluation result: ⊚).

### Example 5

(1) After mixing 0.3 g of bepotastine besilate, 6.66 g of D-mannitol and 0.27 g of hydroxypropyl methylcellulose acetate succinate (grade: HF) in a plastic bag, the mixture was sifted with a 22 mesh sieve (pore size: 710 µm) as defined in the Japanese Pharmacopoeia. 0.5 g of purified water was added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 4.82 g of the aforementioned granulated granules of (1), 1 g of β-cyclodextrin, 0.12 g of crospovidone, 0.06 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 600 kg/pestle) to a weight of 303 mg per tablet to obtain orally disintegrating tablets (hardness: 52 N, oral cavity disintegration time: 38 seconds). In addition, as a result of investigating the ease at which the tablets can be taken with respect to taste, all three subjects did not perceive any bitter taste (bitter taste masking evaluation result: ⊚).

### Example 6

(1) After mixing 0.3 g of bepotastine besilate, 1.5 g of β-cyclodextrin, 6.57 g of D-mannitol and 0.27 g of methacrylic acid copolymer (Eudragit S100) in a plastic bag, the mixture was sifted with a 22 mesh sieve (pore size: 710 µm) as defined in the Japanese Pharmacopoeia. 0.5 g of purified water was added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 2.304 g of the aforementioned granulated granules of (1), 0.048 g of crospovidone, 0.024 g of aspartame and 0.024 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 450 kg/pestle) to a weight of 300 mg per tablet to obtain orally disintegrating tablets (hardness: 51 N, oral cavity disintegration time: 32 seconds). In addition, as a result of investigating the ease at which the tablets can be taken with respect to taste, all three subjects did not perceive any bitter taste (bitter taste masking evaluation result: ⊚).

### Example 7

(1) After mixing 0.3 g of bepotastine besilate, 1.5 g of β-cyclodextrin, 6.57 g of D-mannitol and 0.27 g of carboxymethyl ethyl cellulose (CMEC) in a plastic bag, the mixture was sifted with a 22 mesh sieve (pore size: 710 µm) as defined in the Japanese Pharmacopoeia. 0.5 g of purified water were added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 2.304 g of the aforementioned granulated granules of (1), 0.048 g of crospovidone, 0.024 g of aspartame and 0.024 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 400 kg/pestle) to a weight of 300 mg per tablet to obtain orally disintegrating tablet (hardness: 54 N, oral cavity disintegration time: 34 seconds). In addition, as a result of investigating the ease at which the tablets can be taken with respect to taste, all three subjects did not perceive any bitter taste (bitter taste masking evaluation result: ⊚).

### Example 8

(1) After mixing 0.30 g of bepotastine besilate, 1.5 g of β-cyclodextrin, 6.57 g of D-mannitol and 0.27 g of ethyl cellulose (EC #10) in a plastic bag, the mixture was sifted with a 22 mesh sieve (pore size: 710 µm) as defined in the Japanese Pharmacopoeia. 0.5 g of purified water was added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 2.304 g of the aforementioned granulated granules of (1), 0.048 g of crospovidone, 0.024 g of aspartame and 0.024 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 400 kg/pestle) to a weight of 300 mg per tablet to obtain orally disintegrating tablets (hardness: 55 N, oral cavity disintegration time: 35 seconds). In addition, as a result of investigating the ease at which the tablets can be taken with respect to taste, all three subjects did not perceive any bitter taste (bitter taste masking evaluation result: ⊚).

### Example 9

(1) After mixing 13 g of bepotastine besilate, 65 g of β-cyclodextrin and 288.6 g of D-mannitol in a plastic bag, the mixture was sifted with a 22 mesh sieve (pore size: 710 µm) as defined in the Japanese Pharmacopoeia. The resulting sifted product was charged into a fluidized bed granulator (Powrex Corp., MP-01/03) followed by granulating while spraying with 146.3 g of a 80% aqueous ethanol solution of 8% hydroxypropyl methylcellulose acetate succinate (grade: HF) at a supply air temperature of 50°C over about 30 minutes. The granulation product was dried until the temperature thereof reached 35°C or higher after which the dried product was completely sifted with a 22 mesh sieve (pore size: 710 µm) as defined in the Japanese Pharmacopoeia to obtain granulated granules.
(2) 5.82 g of the aforementioned granulated granules of (1), 0.12 g of croscarmellose sodium, 0.03 g of aspartame and 0.03 g of magnesium stearate were mixed to obtain granules for tableting. The resulting granules for tableting were then compressed with a compaction analyzer (Kikusui Seisakusho Ltd., pestle diameter: 10 mm, tableting pressure: 350 kg/pestle) to a weight of 300 mg per tablet to obtain orally disintegrating tablets (hardness: 52 N, oral cavity disintegration time: 28 seconds). In addition, as a result of investigating the ease at which the tablets can be taken with respect to taste, all three subjects did not perceive any bitter taste (bitter taste masking evaluation result: ⊚).

### Example 10

5.82 g of the granulated granules of Example 1, 0.12 g of croscarmellose sodium, 0.03 g of aspartame and 0.03 g of magnesium stearate were mixed to obtain granules for tableting. The resulting granules for tableting were then compressed with a compaction analyzer (Kikusui Seisakusho Ltd., pestle diameter: 10 mm, tableting pressure: 400 kg/pestle) to a weight of 300 mg per tablet to obtain orally disintegrating tablets (hardness: 55 N, oral cavity disintegration time: 29 seconds). In addition, as a result of investigating the ease at which the tablets can be taken with respect to taste, all three subjects did not perceive any bitter taste (bitter taste masking evaluation result: ⊚).

### Example 11

5.82 g of the granulated granules of Example 1, 0.12 g of croscarmellose sodium, 0.03 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 400 kg/pestle) to a weight of 301.5 mg per tablet to obtain orally disintegrating tablets (hardness: 58 N, oral cavity disintegration time: 31 seconds). In addition, as a result of investigating the ease at which the tablets can be taken with respect to taste, all three subjects did not perceive any bitter taste (bitter taste masking evaluation result: ⊚).

### Example 12

5.82 g of the granulated granules of Example 1, 0.3 g of carboxymethyl cellulose, 0.06 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 400 kg/pestle) to a weight of 312 mg per tablet to obtain orally disintegrating tablets (hardness: 48 N, oral cavity disintegration time: 30 seconds). In addition, as a result of investigating the ease at which the tablets can be taken with respect to taste, all three subjects did not perceive any bitter taste (bitter taste masking evaluation result: ⊚).

### Example 13

(1) After mixing 0.3 g of bepotastine besilate, 1.5 g of β-cyclodextrin, 6.57 g of erythritol and 0.27 g of hydroxypropyl methylcellulose acetate succinate (grade: HF) in a plastic bag, the mixture was sifted with a 22 mesh sieve (pore size: 710 µm) as defined in the Japanese Pharmacopoeia. 0.5 g of purified water was added to the resulting sifted product while granulating, followed by drying for 1 hour at 50°C in a through-circulating box-type dryer and completely sifting the dried product with a 22 mesh sieve (pore size: 710 µm) as defined in the Japanese Pharmacopoeia to obtain granulated granules.
(2) 2.304 g of the aforementioned granulated granules of (1), 0.048 g of crospovidone, 0.024 g of aspartame and 0.024 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 550 kg/pestle) to a weight of 300 mg per tablet to obtain orally disintegrating tablets (hardness: 51 N, oral cavity disintegration time: 31 seconds). In addition, as a result of investigating the ease at which the tablets can be taken with respect to taste, all three subjects did not perceive any bitter taste (bitter taste masking evaluation result: ⊚).

### Example 14

(1) After mixing 17.5 g of bepotastine besilate, 87.5 g of β-cyclodextrin, 279.3 g of D-mannitol and 21 g of hydroxypropyl methylcellulose acetate succinate (grade: HF) in a plastic bag, the mixture was sifted with a 22 mesh sieve (pore size: 710 µm) as defined in the Japanese Pharmacopoeia. The resulting sifted product was charged into a fluidized bed granulator (Powrex Corp., MP-01/03) followed by granulating while spraying with 200 g of purified water at a supply air temperature of 50°C over about 50 minutes. The granulation product was dried until the temperature thereof reached 35°C or higher after which the dried product was completely sifted with a 22 mesh sieve (pore size: 710 µm) as defined in the Japanese Pharmacopoeia to obtain granulated granules.
(2) 347.4 g of the aforementioned granulated granules of (1), 7.2 g of croscarmellose sodium, 3.6 g of aspartame and 1.8 g of magnesium stearate were mixed to obtain granules for tableting. The resulting granules for tableting were then compressed with a rotary tableting machine (Kikusui Seisakusho Ltd., VELA, pestle diameter: 9.5 mm, tableting pressure: 400 kg/pestle) to a weight of 240 mg per tablet to obtain orally disintegrating tablets (hardness: 48 N, oral cavity disintegration time: 27 seconds). In addition, as a result of investigating the ease at which the tablets can be taken with respect to taste, all three subjects did not perceive any bitter taste (bitter taste masking evaluation result: ⊚).

### Example 15

(1) After mixing 17.5 g of bepotastine besilate, 87.5 g of β-cyclodextrin, 258.3 g of D-mannitol and 42 g of hydroxypropyl methylcellulose acetate succinate (grade: HF) in a plastic bag, the mixture was sifted with a 22 mesh sieve (pore size: 710 µm) as defined in the Japanese Pharmacopoeia. The resulting sifted product was charged into a fluidized bed granulator (Powrex Corp., MP-01/03) followed by granulating while spraying with 200 g of purified water at a supply air temperature of 50°C over about 50 minutes. The granulation product was dried until the temperature thereof reached 35°C or higher after which the dried product was completely sifted with a 22 mesh sieve (pore size: 710 µm) as defined in the Japanese Pharmacopoeia to obtain granulated granules.
(2) 347.4 g of the aforementioned granulated granules of (1), 7.2 g of croscarmellose sodium, 3.6 g of aspartame and 1.8 g of magnesium stearate were mixed to obtain granules for tableting. The resulting granules for tableting were then compressed with a rotary tableting machine (Kikusui Seisakusho Ltd., VELA, pestle diameter: 9.5 mm, tableting pressure: 300 kg/pestle) to a weight of 240 mg per tablet to obtain orally disintegrating tablets (hardness: 51 N, oral cavity disintegration time: 25 seconds). In addition, as a result of investigating the ease at which the tablets can be taken with respect to taste, all three subjects did not perceive any bitter taste (bitter taste masking evaluation result: ⊚).

### Example 16

(1) After mixing 17.5 g of bepotastine besilate, 87.5 g of β-cyclodextrin, 230.7 g of D-mannitol and 42 g of hydroxypropyl methylcellulose acetate succinate (grade: HF) in a plastic bag, the mixture was sifted with a 22 mesh sieve (pore size: 710 µm) as defined in the Japanese Pharmacopoeia. The resulting sifted product was charged into a fluidized bed granulator (Powrex Corp., MP-01/03) followed by granulating while spraying with 200 g of purified water at a supply air temperature of 70°C over about 50 minutes. The granulation product was dried until the temperature thereof reached 35°C or higher after which the dried product was completely sifted with a 22 mesh sieve (pore size: 710 µm) as defined in the Japanese Pharmacopoeia to obtain granulated granules.
(2) 48.625 g of the aforementioned granulated granules of (1), 1 g of croscarmellose sodium, 0.250 g of aspartame and 0.125 g of magnesium stearate were mixed to obtain mixed granules. 7 g of the resulting mixed granules and 0.007 g of strawberry essence were then mixed to obtain granules for tableting. The resulting granules for tableting were then compressed with a compaction analyzer (Kikusui Seisakusho Ltd., pestle diameter: 9.5 mm, tableting pressure: 300 kg/pestle) to a weight of 240 mg per tablet to obtain orally disintegrating tablets (hardness: 54 N, oral cavity disintegration time: 19 seconds). In addition, as a result of investigating the ease at which the tablets can be taken with respect to taste, all three subjects did not perceive any bitter taste (bitter taste masking evaluation result: ⊚).

### Example 17

(1) After mixing 17.5 g of bepotastine besilate, 345.8 g of D-mannitol and 42 g of hydroxypropyl methylcellulose acetate succinate (grade: HF) in a plastic bag, the mixture was sifted with a 22 mesh sieve (pore size: 710 µm) as defined in the Japanese Pharmacopoeia. The resulting sifted product was charged into a fluidized bed granulator (Powrex Corp., MP-01/03) followed by granulating while spraying with 200 g of purified water at a supply air temperature of 70°C over about 50 minutes. The granulation product was dried until the temperature thereof reached 35°C or higher after which the dried product was completely sifted with a 22 mesh sieve (pore size: 710 µm) as defined in the Japanese Pharmacopoeia to obtain granulated granules.
(2) After melting 2.16 g of 1-menthol by heating, the 1-menthol was added to 1.26 g of hydrated silicon dioxide (Carplex #80, DSL Japan Co., Ltd.) followed by the addition of 8.82 g of D-mannitol and 0.36 g of peppermint oil to obtain a preliminary corrective preparation.
(3) 6.948 g of the aforementioned granulated granules of (1), 0.252 g of the aforementioned preliminary corrective preparation of (2), 0.144 g of croscarmellose sodium, 0.036 g of aspartame and 0.036 g of magnesium stearate were mixed to obtain granules for tableting. The resulting granules for tableting were then compressed with a compaction analyzer (Kikusui Seisakusho Ltd., pestle diameter: 9.5 mm, tableting pressure: 1250 kg/pestle) to a weight of 240 mg per tablet to obtain orally disintegrating tablets (hardness: 50 N, oral cavity disintegration time: 18 seconds). In addition, as a result of investigating the ease at which the tablets can be taken with respect to taste, all three subjects did not perceive any bitter taste (bitter taste masking evaluation result: ⊚).

### Example 18

(1) 80 g of bepotastine besilate, 1616 g of D-mannitol sifted with a 22 mesh sieve (pore size: 710 µm) as defined in the Japanese Pharmacopoeia and 480 g of hydroxypropyl methylcellulose acetate succinate (grade: HF) were charged into a high-speed mixer granulator (Powrex Corp., FM-VG-10) followed by granulating while introducing 435.2 g of purified water over about 2 minutes followed by granulating for 1 minute. Subsequently, the equipment was cleaned followed by further granulating for 2 minutes to obtain a granulation product. The granulation product was charged into a fluid bed dryer (Freund Industrial Co., Ltd., FLO-5/2SJ) followed by drying at a supply air temperature of 70°C until the product temperature reached 45°C or higher and then completely sifting the dried product with a 42 mesh sieve (pore size: 355 µm) as defined in the Japanese Pharmacopoeia to obtain granulated granules.
(2) 54 g of 1-menthol were charged into a high-speed mixer granulator (Fukae Powtec Co., Ltd., LFS-GS-2J), and after melting by heating at about 80°C, 9 g of peppermint oil and 270 g of D-mannitol were added followed by granulating for 1 minute (granulation product (i)). Subsequently, the equipment was cleaned and 66 g of hydrated silicon dioxide (Carplex #80, DSL Japan Co., Ltd.) were added to granulation product (i) followed by granulating for 1 minute (granulation product (ii)). Granulation product (ii) was then completely sifted using a 22 mesh sieve (pore size: 710 µm) as defined in the Japanese Pharmacopoeia to obtain a preliminary corrective preparation.
(3) 1904 g of the aforementioned granulated granules of (1), 93.1 g of the aforementioned preliminary corrective preparation of (2), 42 g of croscarmellose sodium and 21 g of aspartame were charged into a mixer (Kotobuki Engineering Manufacturing Co., Ltd., LM20) and mixed for 10 minutes (mixed granules (i)).
(4) A portion of the aforementioned mixed granules of (3) and 42 g of sodium stearyl fumarate sifted with a 42 mesh sieve (pore size: 355 µm) as defined in the Japanese Pharmacopoeia were mixed in a plastic bag (mixed granules (ii)). The mixed granules (ii) were charged into a mixer (Kotobuki Engineering Manufacturing Co., Ltd., LM20) and mixed for 2 minutes with the remainder of the mixed particles (i) to obtain granules for tableting. The resulting tablet granules were then compressed with a rotary tableting machine (Kikusui Seisakusho Ltd., VIRG, pestle diameter: 9.5 mm, tableting pressure: 900 kg/pestle) to a weight of 300 mg per tablet to obtain orally disintegrating tablets (hardness: 55 N, oral cavity disintegration time: 26 seconds). In addition, as a result of investigating the ease at which the tablets can be taken with respect to taste, all three subjects did not perceive any bitter taste (bitter taste masking evaluation result: ⊚).

### Example 19

(1) 80 g of bepotastine besilate, 1592 g of D-mannitol sifted with a 22 mesh sieve (pore size: 710 µm) as defined in the Japanese Pharmacopoeia and 480 g of hydroxypropyl methylcellulose acetate succinate (grade: HF) were charged into a high-speed mixer granulator (Powrex Corp., FM-VG-10) followed by granulating while introducing 430.4 g of purified water over about 2 minutes followed by granulating for 1 minute. Subsequently, the equipment was cleaned followed by further granulating for 2 minutes to obtain a granulation product. The granulation product was charged into a fluid bed dryer (Freund Industrial Co., Ltd., FLO-5/2SJ) followed by drying at a supply air temperature of 70°C until the product temperature reached 45°C or higher and then completely sifting the dried product with a 42 mesh sieve (pore size: 355 µm) as defined in the Japanese Pharmacopoeia to obtain granulated granules.
(2) 1883 g of the aforementioned granulated granules of (1), 93.1 g of the preliminary corrective preparation of Example 18, Section (2), 42 g of croscarmellose sodium and 42 g of aspartame were charged into a mixer (Kotobuki Engineering Manufacturing Co., Ltd., LM20) and mixed for 10 minutes (mixed granules (i)).
(3) A portion of the aforementioned mixed granules of (2) and 42 g of sodium stearyl fumarate sifted with a 42 mesh sieve (pore size: 355 µm) as defined in the Japanese Pharmacopoeia were mixed in a plastic bag (mixed granules (ii)). The mixed granules (ii) were charged into a mixer (Kotobuki Engineering Manufacturing Co., Ltd., LM20) and mixed for 2 minutes with the remainder of the mixed particles (i) to obtain granules for tableting. The resulting tablet granules were then compressed with a rotary tableting machine (Kikusui Seisakusho Ltd., VIRG, pestle diameter: 9.5 mm, tableting pressure: 800 kg/pestle) to a weight of about 300 mg per tablet to obtain orally disintegrating tablets (hardness: 56 N, oral cavity disintegration time: 30 seconds). In addition, as a result of investigating the ease at which the tablets can be taken with respect to taste, all three subjects did not perceive any bitter taste (bitter taste masking evaluation result: ⊚).

### Example 20

(1) 80 g of bepotastine besilate, 1640 g of D-mannitol sifted with a 22 mesh sieve (pore size: 710 µm) as defined in the Japanese Pharmacopoeia and 480 g of hydroxypropyl methylcellulose acetate succinate (grade: HF) were charged into a high-speed mixer granulator (Powrex Corp., FM-VG-10) followed by granulating while introducing 440 g of purified water over about 2 minutes followed by granulating for 1 minute. Subsequently, the equipment was cleaned followed by further granulating for 2 minutes to obtain a granulation product. The granulation product was charged into a fluid bed dryer (Freund Industrial Co., Ltd., FLO-5/2SJ) followed by drying at a supply air temperature of 70°C until the product temperature reached 45°C or higher and then completely sifting the dried product with a 42 mesh sieve (pore size: 355 µm) as defined in the Japanese Pharmacopoeia to obtain granulated granules.
(2) 1925 g of the aforementioned granulated granules of (1), 93.1 g of the preliminary corrective preparation of Example 18, Section (2), 42 g of croscarmellose sodium and 21 g of aspartame were charged into a mixer (Kotobuki Engineering Manufacturing Co., Ltd., LM20) and mixed for 10 minutes (mixed granules (i)).
(3) A portion of the aforementioned mixed granules of (2) and 21 g of magnesium stearate sifted with a 42 mesh sieve (pore size: 355 µm) as defined in the Japanese Pharmacopoeia were mixed in a plastic bag (mixed granules (ii)). The mixed granules (ii) were charged into a mixer (Kotobuki Engineering Manufacturing Co., Ltd., LM20) and mixed for 2 minutes with the remainder of the mixed particles (i) to obtain granules for tableting. The resulting tablet granules were then compressed with a rotary tableting machine (Kikusui Seisakusho Ltd., VIRG, pestle diameter: 9.5 mm, tableting pressure: 800 kg/pestle) to a weight of about 300 mg per tablet to obtain orally disintegrating tablets (hardness: 47 N, oral cavity disintegration time: 31 seconds). In addition, as a result of investigating the ease at which the tablets can be taken with respect to taste, all three subjects did not perceive any bitter taste (bitter taste masking evaluation result: ⊚).

### Comparative Example 1

(1) After mixing 0.3 g of bepotastine besilate, 8.16 g of D-mannitol and 0.27 g of hydroxypropyl methylcellulose acetate succinate (grade: HF) in a plastic bag, the mixture was sifted with a 22 mesh sieve (pore size: 710 µm) as defined in the Japanese Pharmacopoeia. After granulating while adding 1 g of 80% EtOH to the resulting sifted product, the granulated product was dried for 1 hour at 50°C in a through-circulating box-type dryer followed by completely sifting the dried product with a 22 mesh sieve (pore size: 710 µm) as defined in the Japanese Pharmacopoeia to obtain granulated granules.
(2) 5.82 g of the aforementioned granulated granules of (1), 0.12 g of croscarmellose sodium and 0.06 g of magnesium stearate were mixed to obtain granules for tableting. The resulting granules for tableting were then compressed with a compaction analyzer (Kikusui Seisakusho Ltd., pestle diameter: 10 mm, tableting pressure: 500 kg/pestle) to a weight of 300 mg per tablet to obtain orally disintegrating tablets (hardness: 50 N, oral cavity disintegration time: 19 seconds). In addition, as a result of investigating the ease at which the tablets can be taken with respect to taste, all three subjects perceived a bitter taste (bitter taste masking evaluation result: ×).

### Comparative Example 2

0.015 g of aspartame was added to 3.0 g of the granules for tableting of Comparative Example 1 and mixed to obtain granules for tableting. The resulting granules for tableting were then compressed with a compaction analyzer (Kikusui Seisakusho Ltd., pestle diameter: 10 mm, tableting pressure: 600 kg/pestle) to a weight of 301.5 mg per tablet to obtain orally disintegrating tablets (hardness: 50 N, oral cavity disintegration time: 20 seconds). In addition, as a result of investigating the ease at which the tablets can be taken with respect to taste, all three subjects perceived a bitter taste (bitter taste masking evaluation result: ×).

### Comparative Example 3

(1) After mixing 0.3 g of bepotastine besilate, 8.16 g of D-mannitol and 0.27 g of ethyl cellulose (EC #10) in a plastic bag, the mixture was sifted with a 22 mesh sieve (pore size: 710 µm) as defined in the Japanese Pharmacopoeia. After granulating while adding 1 g of 80% EtOH to the resulting sifted product, the granulated product was dried for 1 hour at 50°C in a through-circulating box-type dryer followed by completely sifting the dried product with a 22 mesh sieve (pore size: 710 µm) as defined in the Japanese Pharmacopoeia to obtain granulated granules.
(2) 5.82 g of the aforementioned granulated granules of (1), 0.12 g of croscarmellose sodium and 0.03 g of sucralose and 0.06 g of magnesium stearate were mixed to obtain granules for tableting. The resulting granules for tableting were then compressed with a compaction analyzer (Kikusui Seisakusho Ltd., pestle diameter: 10 mm, tableting pressure: 550 kg/pestle) to a weight of 301.5 mg per tablet to obtain orally disintegrating tablets (hardness: 50 N, oral cavity disintegration time: 28 seconds). In addition, as a result of investigating the ease at which the tablets can be taken with respect to taste, all three subjects perceived a bitter taste (bitter taste masking evaluation result: ×).

### Comparative Example 4

(1) After mixing 0.3 g of bepotastine besilate, 6.66 g of D-mannitol, 1.5 g of
   β-cyclodextrin and 0.27 g of hydroxypropyl methylcellulose (HPC-SL) in a plastic bag, the mixture was sifted with a 22 mesh sieve (pore size: 710 µm) as defined in the Japanese Pharmacopoeia. After granulating while adding 0.5 g of purified water to the resulting sifted product, the granulated product was dried for 1 hour at 50°C in a through-circulating box-type dryer followed by completely sifting the dried product with a 22 mesh sieve (pore size: 710 µm) as defined in the Japanese Pharmacopoeia to obtain granulated granules.
(2) 5.82 g of the aforementioned granulated granules of (1), 0.12 g of crospovidone, 0.06 g of aspartame and 0.06 g of sodium stearyl fumarate were mixed to obtain granules for tableting. The resulting granules for tableting were then compressed with a compaction analyzer (Kikusui Seisakusho Ltd., pestle diameter: 10 mm, tableting pressure: 400 kg/pestle) to a weight of 303 mg per tablet to obtain orally disintegrating tablets (hardness: 51 N, oral cavity disintegration time: 57 seconds). In addition, as a result of investigating the ease at which the tablets can be taken with respect to taste, all three subjects did not perceive any bitter taste (bitter taste masking evaluation result: ⊚).

### Comparative Example 5

(1) After mixing 0.3 g of bepotastine besilate, 7.59 g of D-mannitol and 0.27 g of hydroxypropyl methylcellulose (HPC-SL) in a plastic bag, the mixture was sifted with a 22 mesh sieve (pore size: 710 µm) as defined in the Japanese Pharmacopoeia. After granulating while adding 0.8 g of purified water to the resulting sifted product, the granulated product was dried for 1 hour at 50°C in a through-circulating box-type dryer followed by completely sifting the dried product with a 22 mesh sieve (pore size: 710 µm) as defined in the Japanese Pharmacopoeia to obtain granulated granules.
(2) 5.44 g of the aforementioned granulated granules of (1), 0.266 g of the preliminary corrective preparation of Example 18, Section (2), 0.12 g of croscarmellose sodium, 0.06 g of aspartame and 0.12 g of sodium stearyl fumarate were mixed to obtain granules for tableting. Although the resulting granules for tableting were then compressed with a compaction analyzer (Kikusui Seisakusho Ltd., pestle diameter: 9.5 mm, tableting pressure: 1000 kg/pestle) to a weight of 300 mg per tablet to obtain orally disintegrating tablets (hardness: 55 N, oral cavity disintegration time: 105 seconds), disintegration in the mouth was inadequate. In addition, as a result of investigating the ease at which the tablets can be taken with respect to taste, all three subjects did not perceive any bitter taste (bitter taste masking evaluation result: ⊚).

## Claims

1. An orally disintegrating tablet comprising bepotastine or a pharmacologically acceptable salt thereof, menthol or cyclodextrin, a water-insoluble polymer, and a disintegrating agent.

2. The orally disintegrating tablet according to claim 1, wherein the bepotastine or pharmacologically acceptable salt thereof is bepotastine besilate.

3. The orally disintegrating tablet according to claim 1 or claim 2, comprising menthol.

4. The orally disintegrating tablet according to claim 3, wherein the ratio of bepotastine or pharmacologically acceptable salt thereof to menthol is a mass ratio within the range of 1:0.1 to 0.3.

5. The orally disintegrating tablet according to claim 3, comprising 3 to 5 parts by weight of bepotastine or a pharmacologically acceptable salt thereof, 0.1 to 1 part by weight of 1-menthol, 5 to 25 parts by weight of a water-insoluble polymer, 1 to 5 parts by weight of a disintegrating agent, and 65 to 90 parts by weight of a vehicle based on a total of 100 parts by weight of the tablet.

6. The orally disintegrating tablet according to claim 1 or claim 2, comprising cyclodextrin.

7. The orally disintegrating tablet according to claim 5, wherein the cyclodextrin is β-cyclodextrin.

8. The orally disintegrating tablet according to claim 6 or claim 7, wherein the ratio of bepotastine or pharmacologically acceptable salt thereof to cyclodextrin is a mass ratio within the range of 1:3 to 10.

9. The orally disintegrating tablet according to claim 6 or claim 7, wherein the ratio of bepotastine or pharmacologically acceptable salt thereof to cyclodextrin is a mass ratio within the range of 1:4 to 6.

10. The orally disintegrating tablet according to claim 6, comprising 3 to 5 parts by weight of bepotastine or a pharmacologically acceptable salt thereof, 15 to 25 parts by weight of cyclodextrin, 3 to 15 parts by weight of a water-insoluble polymer, 2 to 5 parts by weight of a disintegrating agent, and 55 to 75 parts by weight of a vehicle based on a total of 100 parts by weight of the tablet.

11. The orally disintegrating tablet according to any one of claims 1 to 10, wherein the water-insoluble polymer is selected from the group consisting of hydroxypropyl methylcellulose acetate succinate, methacrylic acid copolymer S, carboxymethyl ethyl cellulose and ethyl cellulose.

12. The orally disintegrating tablet according to any one of claims 1 to 10, wherein the water-insoluble polymer is hydroxypropyl methylcellulose acetate succinate.

13. The orally disintegrating tablet according to any one of claims 1 to 12, wherein the disintegrating agent is selected from the group consisting of croscarmellose sodium, carboxymethyl cellulose and crospovidone.

14. The orally disintegrating tablet according to any one of claims 1 to 12, wherein the disintegrating agent is croscarmellose sodium or carboxymethyl cellulose.

15. The orally disintegrating tablet according to any one of claims 1 to 14, further comprising a vehicle.

16. The orally disintegrating tablet according to claim 15, wherein the vehicle is a sugar-alcohol or sugar.

17. The orally disintegrating tablet according to any one of claims 1 to 16, wherein the oral cavity disintegration time is within 50 seconds.

18. The orally disintegrating tablet according to any one of claims 1 to 17, wherein the hardness is 25 to 60 N.

19. A method for producing an orally disintegrating tablet, comprising:
1) a step for obtaining granulated granules by mixing and granulating bepotastine or a pharmacologically acceptable salt thereof, a water-insoluble polymer, and optionally a vehicle,
2) a step for obtaining granules for tableting by mixing the granulated granules, menthol, a disintegrating agent, and optionally a lubricant and/or sweetener, and
3) a step for compressing the granules for tableting.

20. A method for producing an orally disintegrating tablet, comprising:
1) a step for obtaining granulated granules by mixing and granulating bepotastine or a pharmacologically acceptable salt thereof, cyclodextrin, a water-insoluble polymer, and optionally a vehicle,
2) a step for obtaining granules for tableting by mixing the granulated granules, a disintegrating agent, and optionally a lubricant and/or sweetener, and
3) a step for compressing the granules for tableting.

21. An orally disintegrating tablet which comprises incorporating granulated granules obtained by mixing and granulating bepotastine or a pharmacologically acceptable salt thereof, a water-insoluble polymer, and optionally a vehicle.

22. An orally disintegrating tablet which comprises incorporating granulated granules obtained by granulating bepotastine or a pharmacologically acceptable salt thereof and a water-insoluble polymer by a wet granulation method.

23. Granulated granules obtained by granulating bepotastine or a pharmacologically acceptable salt thereof and a water-insoluble polymer by a wet-granulation method.
